# EUROPÄISCHE PATENTSCHRIFT

(11) **EP 4 558 102 B1**
(45) Veröffentlichungstag und Bekanntmachung des Hinweises auf die Patenterteilung: **02.09.2026**
(21) Anmeldenummer: 23744387.4
(22) Anmeldetag: 11.07.2023
(51) Int. Cl.: A61F 2/76

(54) **VERFAHREN UND COMPUTERPROGRAMM ZUM ERSTELLEN EINES DIGITALEN 3D-MODELLS SOWIE VERFAHREN ZUM EINSTELLEN ODER HERSTELLEN EINER ORTHOPÄDIETECHNISCHEN VERSORGUNG**
METHOD AND COMPUTER PROGRAMME FOR CREATING A DIGITAL 3D MODEL, AND METHOD FOR ADJUSTING OR PRODUCING AN ORTHOPAEDIC DEVICE
PROCÉDÉ ET PROGRAMME INFORMATIQUE POUR CRÉER UN MODÈLE 3D NUMÉRIQUE, ET PROCÉDÉ POUR AJUSTER OU PRODUIRE UN DISPOSITIF ORTHOPÉDIQUE

(30) Priorität: 15.07.2022 DE 102022117757
(43) Veröffentlichungstag der Anmeldung: 28.05.2025
(73) Patentinhaber: Ottobock SE & Co. KGaA, 37115 Duderstadt (DE)
(72) Erfinder: MOUROUM, Marvin, 37115 Duderstadt (DE)
(74) Vertreter: Gramm, Lins & Partner Patent- und Rechtsanwälte PartGmbB
(86) Internationale Anmeldenummer: PCT/EP2023/069184
(87) Internationale Veröffentlichungsnummer: WO 2024/013166

(56) Entgegenhaltungen:
- WO-A1-2021/008892

## Beschreibung

Die Erfindung betrifft ein Verfahren zum Erstellen eines digitalen 3D-Modells eines Körperteils eines Patienten, das wenigstens einen ersten Körperteilabschnitt und wenigstens einen an dem ersten Körperteilabschnitt angrenzenden gelenkigen zweiten Körperteilabschnitt umfasst, sodass der erste Körperteilabschnitt mittels des gelenkigen zweiten Körperteilabschnitts bewegbar ist. Die Erfindung betrifft ebenso ein Computerprogramm hierzu.

Die Erfindung betrifft außerdem ein Verfahren zum Einstellen oder Herstellen einer orthopädietechnischen Versorgung.

Orthopädietechnische Versorgungen (auch orthopädietechnische Vorrichtung oder Einrichtung genannt) sind im Sinne der vorliegenden Erfindung insbesondere Orthesen, Prothesen, Exoskelette und ggf. auch Rollstühle. Orthesen sind Produkte, die ein Körperteil des Patienten, beispielsweise ein Gelenk, stützen, unterstützen, schützen oder in der Bewegungsfreiheit einschränken, um eine Überbeanspruchung zu vermeiden. Prothesen hingegen ersetzen nicht oder nicht mehr vorhandene Körperteile des Patienten. Exoskelette sind insbesondere mechanische Stützstrukturen, die den Hauptbewegungsapparat des Patienten stützen, unterstützen oder schützen sollen.

Unter einem Patienten wird im Folgenden jeder Verwender der orthopädietechnischen Versorgung verstanden. Es handelt sich folglich um den Träger der orthopädietechnischen Versorgung.

Jede orthopädietechnische Versorgung wird an einem Körperteil des Patienten angeordnet. Dabei muss es nicht zwangsläufig mit der Haut des Patienten in Kontakt kommen. Orthesen und Exoskelette werden beispielsweise häufig über der Kleidung getragen, sodass diese, beispielsweise eine Hose, sich zwischen der orthopädietechnischen Versorgung und der Haut des Patienten befindet. Dennoch wird beispielsweise eine Knie-Orthese am Knie oder am Bein des Patienten befestigt. Eine Prothese verfügt immer über ein mit einem Amputationsstumpf oder einem anderen Körperteil verbundenes Schnittstellenelement, das an dem jeweiligen Körperteil befestigt wird. Bei einer Beinprothese wird beispielsweise ein Prothesenschaft verwendet, der die Schnittstelle zwischen der Prothese und dem Amputationsstumpf darstellt. In diesem Fall wäre der Amputationsstumpf das Körperteil des Patienten. Zwischen der Hautoberfläche des Amputationsstumpfes des Patienten und dem Prothesenschaft wird in der Regel ein Liner verwendet, um auf die Haut wirkende Scherkräfte zu reduzieren.

Ein Prothesenschaft für einen Amputationsstumpf wird in der Regel aus einem starren (kaum verformbaren) Material, beispielsweise einem faserverstärkten Kunststoff, hergestellt und bildet einen wichtigen Teil des Interfaces zwischen dem Amputationsstumpf und der Prothese, die am Prothesenschaft angeordnet wird. Insbesondere für Beinprothesen, die an einem Amputationsstumpf, beispielsweise einem Oberschenkelstumpf, angeordnet werden sollen, werden entsprechende Prothesenschäfte seit langem verwendet.

Insbesondere Prothesen schäfte für Bein am putierte sind im täglichen Gebrauch besonderen Belastungen ausgesetzt. Beim Gehen lastet das gesamte Gewicht des Patienten auf dem Prothesenschaft und damit insbesondere auf dem Amputationsstumpf, der in dem Prothesenschaftangeordnet wird. Es ist dah er von größter Wichtigkeit, den Prothesenschaft möglichst optimal an die individuellen Gegebenheiten und Bedürfnisse des Patienten anzupassen, insbesondere an die Form und Geometrie des betreffenden Körperteils.

Um eine orthopädietechnische Versorgung optimal an die Gegebenheiten des Patienten anpassen und einstellen zu können, und zwar insbesondere im Zusammenhang mit einer digitalen Datenverarbeitungsanlage, ist es notwendig, dass zuvor ein möglichst hochgenaues Modell des Körperteils erstellt wird, dass zumindest teilweise mit der orthopädietechnischen Versorgung in Kontakt kommt und mit dieser versorgt werden soll.

In der Praxis istes hierfürbekannt, mith ilfe einer Scan vorrichtung das betrachtete Körperteil zu scannen und hieraus ein digitales 3D-Modell des modellierenden Körperteils zu erstellen. Hierfür können beispielsweise kamerabasierte Bilddaten von dem Körperteil aufgenommen werden und anschließend basierend auf diesen Bilddaten dann ein Modell des Körperteils in der digitalen Datenverarbeitungsanlage zu erstellen. Basierend auf dem digitalen 3D-Modell kann dann beispielsweise die orthopädietechnische Versorgung eingerichtet werden oder ein physisches Modell erstellt werden.

Hält der Patient während des Scanvorgangs ausreichend still, so ist die kamerabasierte Erfassung und Erstellung des 3D-Modells in der Regel unproblematisch. Häufig sind Patienten jedoch nicht in der Lage, das betreffende Körperteil so ausreichend stillzuhalten, dass ein Scanvorgang mit einem guten Ergebniszu Endegeführtwerden kann. Das ist insbesondere in den Fällen, in denen das Körperteil lastenfrei und ohne Haltevorrichtung vermessen werden soll.Auch für Kinder oder Patienten mit Spastiken kann es schwierig bis unmöglich werden, das Körperteil ausreichend ruhig zu halten.

Wird beispielsweise ein Ober- und ein Unterschenkel vermessen, kann das Beugen des Kniegelenkes, das zwischen dem Ober- und Unterschenkel sitzt, zum Abbruch des Scan vorgangs führen, da sich das gesamte Körperteil bestehend aus Unterschenkel, Knie und Oberschenkel deutlich verformt. Der Rekonstruktionsalgorithmus ist dann nicht mehr in der Lage, die einzelnen Bildpunkte entsprechend zuzuordnen und muss so zum Abbruch gebracht werden.

Die WO 2021 / 008 892 A1 offenbart die Erstellung einer Knieprothese, die bei einer Knieoperation in das Knieeingesetztwerden soll. Hierfürwerden, um die notwendigen kinematischen Daten zu erhalten, unterschiedlichste Scanverfahren eingesetzt. Scandaten vom zu operierenden Knie sowie dem Ober- und Unterschenkel werden mit Scanverfahren wie bspw. Röntgen, CT oder MRT erstellt. Es werden außerdem kinematische Daten erstellt, welche die Bewegung des Knies repräsentieren, was mittels einer Kamera und Markern am Patienten erfolgt.

Es ist daher Aufgabe der vorliegenden Erfindung ein verbessertes Verfahren zum Erstellen eines digitalen 3D-Modells eines Körperteils anzugeben, dass mit einer orthopädietechnischen Versorgung versorgt bzw. behandelt werden soll.

Die Aufgabe wird mit dem Verfahren gemäß Anspruch 1 erfindungsgemäß gelöst. Vorteilhafte Ausgestaltungen der Erfindung finden sich dann in den entsprechenden Unteransprüchen.

Gemäß Anspruch 1 wird ein Verfahren zum Erstellen eines digitalen 3D-Modells eines Körperteils eines Patienten vorgeschlagen, wobei das Körperteil wenigstens einen ersten Körperteilabschnitt hat, der an wenigstens einen gelenkigen zweiten Körperteilabschnitt angrenzt, sodass der erste Körperteilabschnitt mittels des gelenkigen zweiten Körperteilabschnitts bewegbar ist. An dem gelenkigen zweiten Körperteilabschnitte können dabei mehr als ein erster Körperteilabschnitt, der insbesondere starr ist, angeordnet sein.

Das Verfahren umfasst dabei die durch eine digitale Datenverarbeitungseinrichtung ausgeführten Schritte:
- Bereitstellen von digitalen, kamerabasierten Bilddaten, die das digital zu modellierende Körperteil des Patienten aus verschiedenen Aufnahmerichtungen beinhalten,
- Identifizieren des wenigstens einen ersten Körperteilabschnitts aus den bereitgestellten digitalen Bilddaten und Erstellen eines ersten 3D-Körperteilabschnittsmodell für den identifizierten ersten Körperteilabschnitt basierend auf den bereitgestellten digitalen Bilddaten und hierzu bereitgestellten Tiefeninformationen des identifizierten ersten Körperteilabschnitts,
- Identifizieren des wenigstens einen gelenkigen zweiten Körperteilabschnitts aus den bereitgestellten digitalen Bilddaten, und
- Erstellen des digitalen 3D-Modells des zu modellierenden Körperteils in Abhängigkeit von dem erstellten ersten 3D-Körperteilabschnittsmodell und dem identifizierten gelenkigen zweiten Körperteilabschnitts.

Demnach wird erfindungsgemäß vorgeschlagen, dass die einzelnen Körperteilabschnitte separat identifiziert und dann basierend auf der separaten Identifikation der Körperteilabschnitte dann das Körperteilabschnitte Modell für zumindest den ersten Körperteilabschnitt erstellt wird, sodass basierend darauf dann das 3D-Modell des zu modellieren Körperteils erstellt werden kann. Das zu modellierende Körperteil wird dabei in einzelnen Körperteilabschnitte unterteilt, da diese einzelnen Körperteilabschnitte für sich genommen nur wenig ihre Form verändern. Die so identifizierten Körperteilabschnitte werden dann später rekonstruiert und zusammengesetzt, wobei hierbei der gelenkige Körperteilabschnitte nicht zwingend Teil des Modells sein muss. Dabei kann jeder starre Körperteilabschnitte unabhängig von der Beugung des Gelenkes für sich genommen rekonstruiert und anschließend unter Bezugnahme des Gelenkes zu einem Gesamtmodell zusammengesetzt werden.

Mit der vorliegenden Erfindung wird es somit möglich, ein 3D-Modell auch dann zu erstellen, wenn der Patient sich bewegt, was insbesondere beim Scannen von Kindern einem großen Vorteil hat, da diese selten in einer ruhigen Position verweilen.

Die Tiefeninformationen können dabei beispielsweise aus den bereitgestellten digitalen Bilddaten ermittelt werden, wenn diese verschiedenen Aufnahmerichtungen des Körperteils beinhalten. Denkbar ist aber auch, dass diese tiefen Informationen aus einer anderen Aufnahmequelle stammen, beispielsweise mittels eines Laserscanners direkt und vorzugsweise zusätzlich zu den Kameradaten erfasst werden.

Gemäß einer Ausführungsform ist vorgesehen, dass ein zweites 3D-Körperteilabschnittsmodell für den identifizierten gelenkigen zweiten Körperteilabschnitt basierend auf den bereitgestellten digitalen Bilddaten und hierzu bereitgestellten Tiefeninformationen des identifizierten gelenkigen zweiten Körperteilabschnitts erstellt wird, wobei das digitale 3D-Modell des zu modellierenden Körperteils in Abhängigkeit von dem erstellten ersten 3D-Körperteilabschnittsmodell und dem erstellten zweiten 3D-Körperteilabschnittsmodell erstellt wird.

Hierbei wird vorteilhafterweise auch für den gelenkigen zweiten Körperteilabschnitt, der zuvor identifiziert wurde, ein Körperteilabschnittsmodell erstellt, sodass sowohl für das oder die ersten Körperteilabschnitte als auch für den gelenkigen zweiten Körperteilabschnitt jeweils ein Körperteilabschnittsmodell erstellt wird, wobei basierend auf diesen Körperteilabschnittsmodellen dann das digitale 3D-Modell erstellt wird. In dieser Ausführungsform ist der gelenkige Körperteilabschnitte Teil des 3D-Modells.

Gemäß einer Ausführungsform ist vorgesehen, dass wenigstens zwei Körperteilabschnitte weiterhin in Abhängigkeit von einer Beziehung zueinander identifiziertwerden.

Unter Kenntnis der Beziehungen der einzelnen Körperteilabschnitte zueinander können aus den digitalen Bilddaten die einzelnen Körperteilabschnitte identifiziert und anschließend das mindestens eine Körperteilabschnittsmodell erstellt werden.

Gemäß einer Ausführungsform ist vorgesehen, dass der gelenkige zweite Körperteilabschnitt in Abhängigkeit von einem Bewegungsraum des Gelenkes oder dass der erste Körperteilabschnitt in Abhängigkeit von einem Bewegungsraum eines an dem ersten Körperteilabschnitt angrenzenden Gelenkes oderdes identifizierten gelenkigen zweiten Körperteilabschnittes identifiziert wird.

Unter Kenntnis des Bewegungsraumes des Gelenkes wird es möglich, die an dem Gelenk angeordneten ersten Körperteilabschnitte, die insbesondere starr ein können, zu identifizieren, um das Körperteilabschnittsmodell für den jeweils identifizierten Körperteilabschnitte zu erstellen. Unter Kenntnis des Bewegungsraumes des Gelenkes wird somit eine mögliche Bewegung des angrenzenden ersten Körperteilabschnittes vorhersagbar, wodurch die Identifikation verbessert wird. Aber auch das Gelenkselber kann unter Kenntnis des Bewegungsraumes besser identifiziert werden. Auch kann die Rekonstruktion hierdurch verbessert werden, da mögliche Abweichungen von der initialen Stellung zu Scanbeginn vorhersagbar werden. Bei Abweichungen, die nicht mehr durch den Bewegungsraum des Gelenkes zu begründen sind, muss es sich dagegen um Artefakte handeln, die verworfen werden können.

Gemäß einer Ausführungsform ist vorgesehen, dass die Körperteilabschnitte des zu modellierenden Körperteils in einem gemeinsamen Scanvorgang erfasst werden, wobei das mindestens eine Körperteilabschnittsmodell separat erstellt wird.

Hierbei wird das gesamte Körperteil, welches in einem 3D-Modell modelliert werden soll, in einem gemeinsamen Scanvorgang erfasst, wobei jedoch die einzelnen Körperteilabschnitte separat identifiziertund die jeweiligen Körperteilabschnittsmodelleseparat erstellt werden. Zur Erstellung des 3D-Modells werden dann die einzelnen Körperteilabschnittsmodelle, die separat erstellt wurden, zu einem gemeinsamen Modell zusammengefügt, um sodass zu modellierende Körperteil zu rekonstruiert.

Gemäß einer Ausführungsform ist vorgesehen, dass ein maschinelles Lernsystem bereitgestellt wird, welches eine Korrelation von digitalen Bilddaten als Eingabedaten mit der jeweiligen Identifikation des entsprechenden Körperteilabschnittes als Ausgabedaten gelernt hat, wobei zur Identifikation die bereitgestellten Bilddaten als Eingabedaten in das maschinelle Lernsystem eingegeben werden und daraufhin eine Identifikation des entsprechenden Körperteilabschnittes als Ausgabedaten erhalten wird.

Das maschinelle Lernsystem, dass in einer bevorzugten Ausführungsform ein künstliches neuronales Netzsein kann, erhältals Eingabedaten diedigitalen Bilddaten, wobei als Ausgabe die Identifikation eines der Körperteilabschnitte erhalten wird. Die Identifikation kann dabei beispielsweise derart erfolgen, dass eine sogenannte Bounding-Box um den identifizierten Körperteilabschnitt gelegt wird, wobei diese Bounding-Box den Umrissen des jeweiligen Körperteilabschnitte entspricht. Denkbar ist aber auch, dass der zu identifizierende Körperteilabschnitte in den digitalen Bilddaten mit einer bestimmten Farbe eingefärbt wird, wobei als Ausgabe des maschinellen Lernsystems Bilddaten erhältlich sind, welche den identifizierten Körperteilabschnitt in einer vorgegebenen Farbe eingefärbtbeinhalten. Letztlich wird durch das masch inelle Lernsystem durch Eingabe der digitalen Bilddaten als Eingabedaten eine Kennung für die Identifikation des entsprechenden Körperteilabschnittes als Ausgabedaten ermittelt, wobei diese Kennung insoweit die Identifikation des Körperteilabschnittes innerhalb der Bilddaten beinhaltet.

Ein Trainingsdatensatz zum Trainieren des maschinellen Lernsystems beinhaltet dabei digitale Bilddaten, denen jeweils eine Kennung für die Identifikation des jeweiligen Körperteilabschnittes zugeordnet sind. D. h., jedem Satz Bilddaten ist eine entsprechende zu lernende Identifikation des Körperteilabschnittes zugeordnet. Zum Trainieren des maschinellen Lern systems wird dabei eine Vielzahl von Trainingsdatensätzen bereitgestellt, um so das maschinelle Lernsystem entsprechend zu konditionieren.

Gemäß einer Ausführungsform ist vorgesehen, dass die digitalen, kamerabasierten Bilddaten kontinuierlich derdigitalen Datenverarbeitungseinrichtung bereitgestelltwerden.

Durch das kontinuierliche Bereitstellen der Bilddaten kann in einer bevorzugten Ausführungsform auch kontinuierlich das 3D-Modell des Körperteils erstellt werden.

Gemäß einer Ausführungsform ist vorgesehen, dass mittels eines mobilen Bildsensors die digitalen, kamerabasierten Bilddaten des zu modellierenden Körperteils des Patienten aufgenommen und der digitalen Datenverarbeitungseinrichtung zum Erstellen des 3D-Körperteilmodells bereitgestellt werden. Ein solcher mobiler Bildsensor kann dabei beispielsweise ein handgeführter Bildsensor sein.

Die Aufgabe wird im Übrigen auch mit dem Computerprogramm gemäß Anspruch 12 erfindungsgemäß gelöst, wobei das Computerprogramm Programmcodemitteln aufweist, die eingerichtet sind, das vorstehend beschriebene Verfahren auszuführen, wenn das Computerprogramm auf einer Datenverarbeitungsanlage ausgeführt wird.

Die Aufgabe wird im Übrigen auch mit dem Verfahren zum Einstellen oder Herstellen einer orthopädietechnischen Versorgung gemäß Anspruch 13 erfindungsgemäß mit den Schritten gelöst:
- Erstellen eines 3D-Körperteilmodells des Körperteils, dass mit der orthopädietechnischen Versorgung ausgestattet werden soll, gemäß dem vorstehend beschriebenen Verfahren, und
- Einstellen oder Herstellen der orthopädietechnischen Versorgung unterVerwendung des 3D-Körperteilmodells.

Die Erfindung wird anhand der beigefügten Figuren beispielhaft erläutert. Es zeigen:
- Figur 1: schematische Darstellung der erfindungsgemäßen Vorrichtung;
- Figur 2: schematische Darstellung eines Scanvorgangs;
- Figur 3: schematische Darstellung einer Rekonstruktion nach den Scannen;
- Figur 4: beispielhafte Darstellung von Bewegungen.
- Figur 5: Darstellung eines weiteren Ausführungsbeispiels

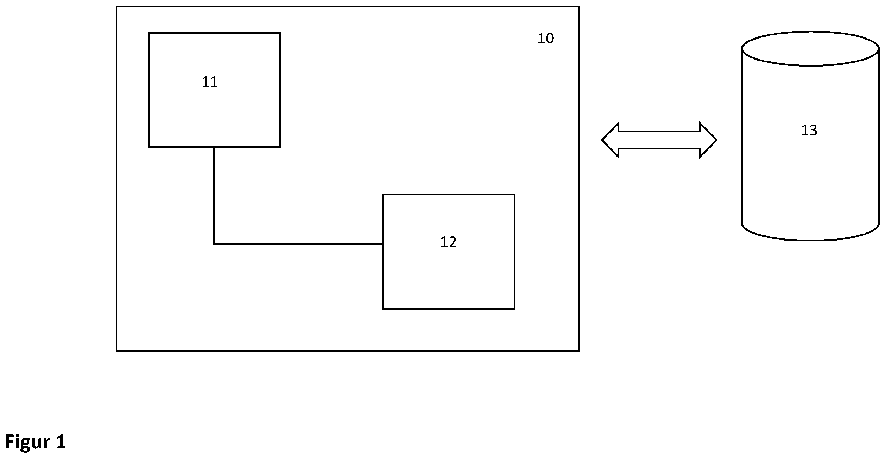

Figur 1 zeigt in einer schematisch stark vereinfachten Darstellung ein mobiles Endgerät 10, dass eine Scanvorrichtung 11 und eine Datenverarbeitungseinrichtung 12 hat. Mithilfe der Scanvorrichtung 11 können digitale Bilddaten des zu modellierenden Körperteils erfasst und an die Datenverarbeitungseinrichtung 12 übertragen werden, um das 3D-Modell des zu modellierende Körperteil zu erstellen. Ein solches 3D-Modell, das erstellt wurde, kann dann beispielsweise in einem digitalen Datenspeicher 13 hinterlegt werden.

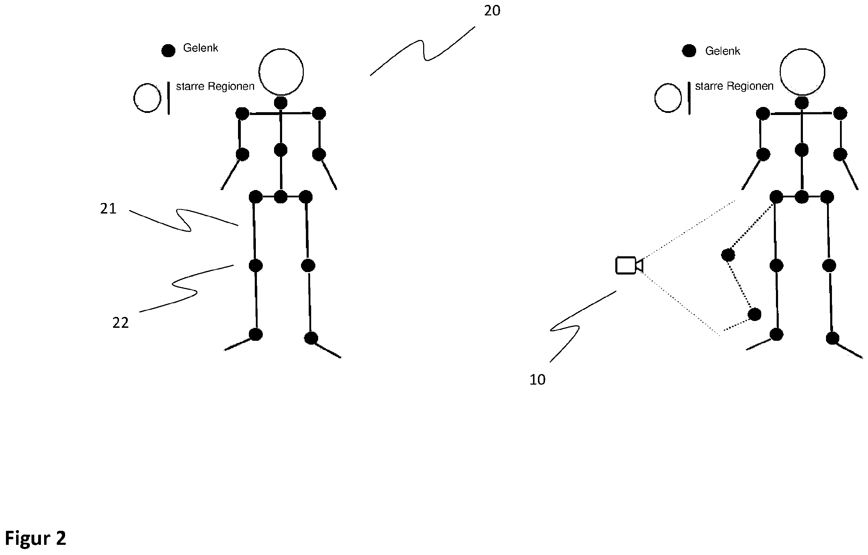

Figur2 zeigt auf der linken Seite eine abstrakte Darstellung einer Person 20, die starre Regionen 21 als erste Körperteilabschnitte und Gelenke 22 als zweite Körperteilabschnitte hat. Die auf der linken Seite gezeigte abstrakte Darstellung der Person 20 zeigt dabei eine Ausgangsposition, bei der die Person 20 keinerlei Bewegung aufweist

Mithilfe eines mobilen Endgerätes 10 soll nun der rechte Ober- und Unterschenkel der Person 20 erfasst und modelliert werden. Dabei kann es passieren, dass die Person 20 ihren Ober- und/oder Unterschenkel bewegt, da sowohl der Oberschenkel an dem Hüftgelenk bewegbar angeordnet ist als auch der Unterschenkel an dem Kniegelenk gegenüber dem Oberschenkel beweglich ist.

Eine solche Bewegung, wie sie auf der rechten Seite der Figur 2 gezeigt ist, die während eines Scan vorgangs geschieht, würde normalerweise zu einem Abbruch des Vorgangs führen, da die Bilderkennung die sich relativ zueinander bewegenden Abschnitte des Körperteils nicht mehr zuordnen können.

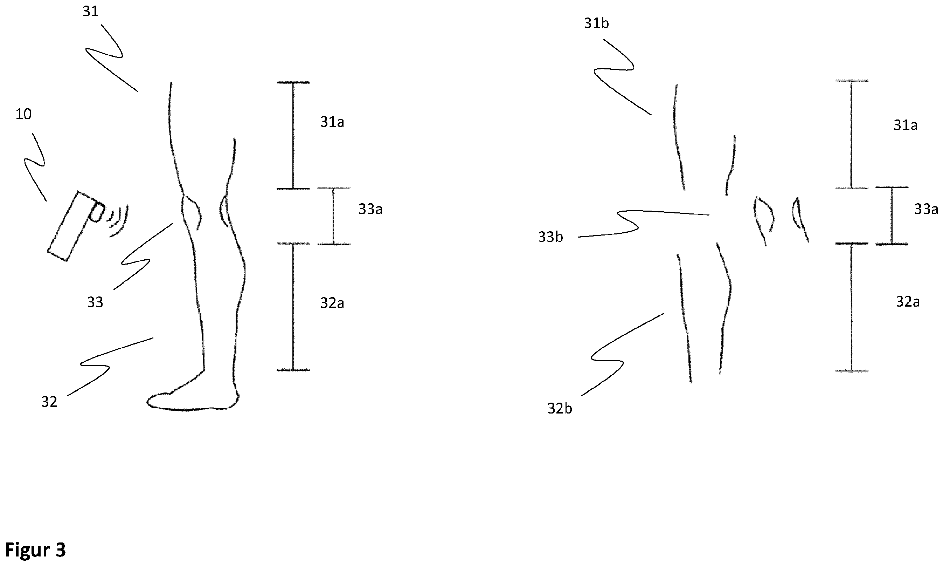

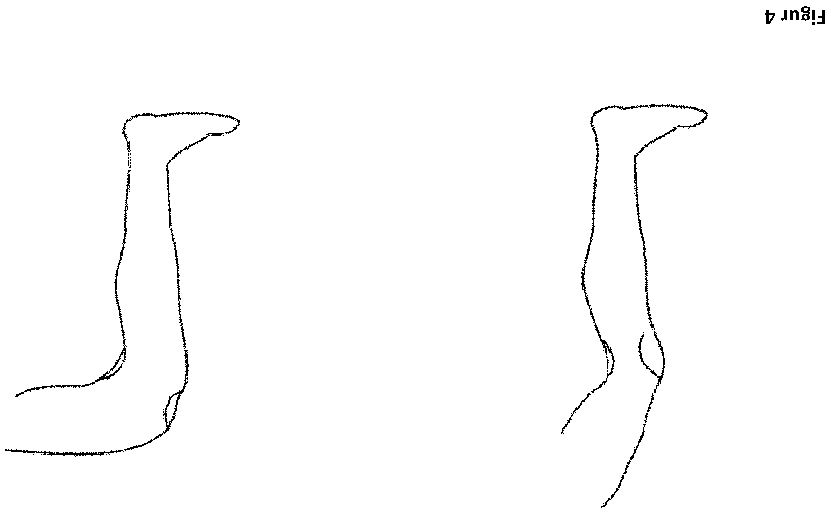

Figur3 zeigt in einerschematisch stark vereinfachten Darstellung einen solchen Scanvorgang. Zunächst wird mithilfe eines mobilen Endgerätes 10 das zu modellierende Körperteil 30 gescannt. Dabei wird mithilfe eines maschinellen Lern systems, welches in dem mobilen Endgerät 10 ausgeführt wird, anhand der Kamera basierten Bilddaten, die beim Scannen erfasst werden, der Unterschenkel, der Oberschenkel sowie das dazwischenliegende Kniegelenk separat und einzelnen identifiziert. Unabhängig von der Beugung des Knies, wie in Figur 4 gezeigt, werden die drei Körperteilabschnitte identifiziert.

Der Oberschenkel 31 und der Unterschenkel 32 bilden dabei die ersten Körperteilabschnitte, die insbesondere starr sind. Dies bedeutet, dass der Oberschenkel 31 und der Unterschenkel 32 insbesondere nicht ein weiteres Gelenk aufweisen, um die grundsätzliche Form zu verändern. Selbst verständlich kann auch bei starren Körperteilabschnitten Weichgewebe vorhanden sein, welches zu einerkurzfristigen Formver-änderung durch äußere Krafteinwirkung neigt.

Das zwischen dem Oberschenkel 31 und dem Unterschenkel 32 vorhandene Kniegelenk 33 führtzu der Möglichkeit, dass sich der Oberschenkel 31 und der Unterschenkel 32 relativ zueinander bewegen lassen. Das Kniegelenk33 bildet im Ausführungsbeispiel der Figur 3 somit den gelenkigen zweiten Körperteilabschnitt.

Ein solcher Scanvorgang ist dabei auf der linken Seite der Figur 3 gezeigt. Dabei werden die drei Körperteilabschnitte 31a bis 33a identifiziert. Anschließend wird basierend auf dieser Identifikation der drei Körperteilabschnitte 31a bis 33a in den digitalen Bilddaten separat jeweils ein Körperteilabschnittsmodell 31b bis 33b für den jeweiligen Körperteilabschnitt 31a bis 33a erstellt.

Nachdem separaten Erstellen der einzelnen Körperteilabschnittsmodelle 31 b bis 33b kann dann basierend auf diesen Abschnittsmodellen dann das gewünschte 3D-Modell erstellt werden.

Basierend auf dem erstellten 3D-Modell kann dann eine orthopädietechnische Versorgung eingestellt werden. Dies umfasst insbesondere das Erstellen der orthopädietechnischen Versorgung basierend auf dem 3D-Modell sowie das Konfigurieren der orthopädietechnischen Versorgung basierend auf dem 3D-Modell.

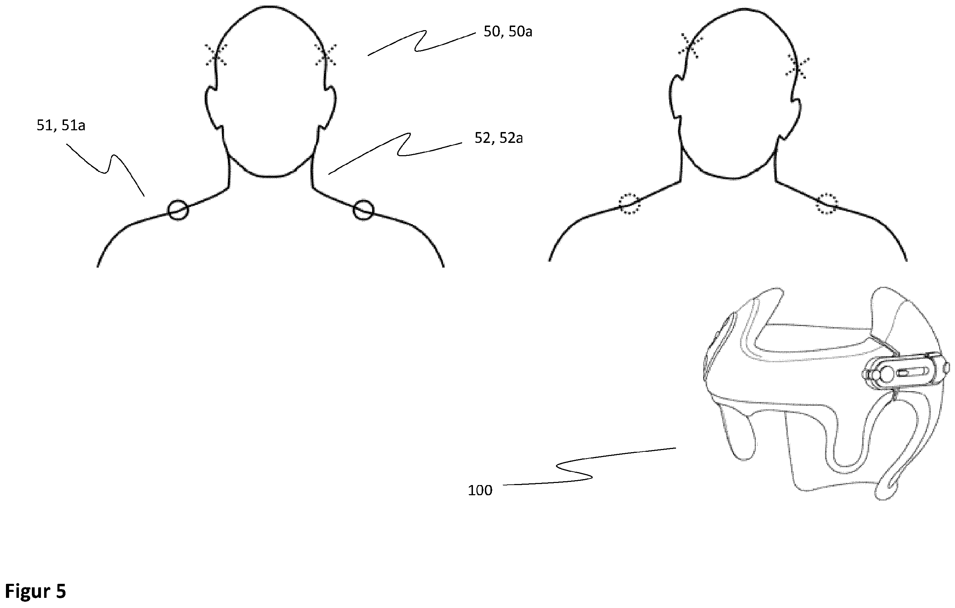

Figur 5 zeigt ein Ausführungsbeispiel, bei dem ein Kopf 50 zur Anfertigung einerorthopädietechnischen Versorgung 100 gescannt werden soll. Der erste Körperteilabschnitt 50a ist dabei starr und weist keine Gelenke auf. Auch ein weiterer erster Körperteilabschnitt51a, der Schulterbereich 51, ist für den Scanvorgang als starrer Körperbereich anzusehen. Zwischen dem Kopf 50 und dem Schulterbereich 51 befindet sich der Hals 52, der ein Gelenk im Sinne der vorliegenden Erfindung darstellt und daher als zweiter Körperteilabschnitt52a angesehen werden kann. Der Hals 52 verbindet dabei den Kopf 50 mit dem Schulterbereich 51 derart, dass der Kopf 50 gegenüber dem Schulterbereich 51 eine Relativbewegung ausführen kann.

Soll beispielsweise der Kopf 50 eines Kindes gescannt werden, so ist es schwierig, den Scanbereich von vornherein auf den Kopf zu beschränken, da in aller Regel sowohl Schultern alsauch Halsmitgescanntwerden. Da das Kind den Kopf während des Scanvorgangs aber meist bewegt, wird der Scann abgebrochen werden oder zu Artefakten führen. Dies kann vermieden werden, indem der Gelenkbereich, der Hals 52, erkannt und die Rekonstruktion auf den starren Abschnitt, also den Kopf 50, begrenzt wird.

Im Ausführungsbeispiel derFigur5 soll eine Kopforthese 100 erstellt werden, die beispielsweise zur Behandlung von Plagiocephalie bei Babys genutzt werden. Hierzu muss der Kopf 50, genauer der obere Bereich des Kopfes 50, gescannt werden. Zwar befinden sich dort keine Gelenke und es handeltsich daher um einen starren Körperteilabschnitt. Allerdings können diese sich beispielsweise überdas Halsgelenk52 als Ganzes bewegen. Zudem ist es kaum möglich, nur den Kopf 50 zu Scannen, da der Scanner meist auch den Hals 52 und die Schultern 51 miterfassen würde.

Die mit X markierten Punkte in Figur5 im oberen Kopfbereich befinden sich in einem starren ersten Körperteilabschnitt50a. Ihre Lage zueinander bleibtauch bei Bewegung des Kopfes 50 gleich. Auch die beiden durch ein O markierten Punkte im Schulterbereich ändern ihre Lage zueinander nicht.

Legt der Patienten jedoch den Kopf schief, sowie dies auf der rechten Seite der Figur 5 gezeigt ist, so ändert sich die Lage der mit X gekennzeichneten Punkte im Kopfbereich zu denen mit einem O gekennzeichneten Punkte im Schulterbereich, was normalerweisezu Artefakten führt und bei der Rekonstruktion oder beim Scanvorgang zu einem Abbruch führen kann.

Erfindungsgemäß wird das Problem hierdurch gelöst, dass der Hals 52 als gelenkiger Bereich erkannt und dadurch der Kopf 50 und Schulterbereich 52 jeweils einzelnen unabhängig voneinander als starre Bereiche rekonstruiert werden.

Im Ausführungsbeispiel der Figur 5 reicht für die Kopforthese 100 die Rekonstruktion des Kopfes 50 aus, sodass der Schulterbereich 52 verworfen werden kann. Es wird daher nur für den Kopf 50 ein entsprechendes Modell erstellt.

### Bezugszeichenliste

10 mobiles Handgerät
11 Scanvorrichtung
12 Datenverarbeitungseinrichtung
13 digitale Datenspeicher
20 Person
21 Oberschenkel/erster Körperteilabschnitt
22 Kniegelenk/zweiter Körperteilabschnitt
31 Oberschenkel
32 Unterschenkel
33 Kniegelenk
31a erster Körperteilabschnitt als Oberschenkel
32a erster Körperteilabschnitte als Unterschenkel
33a zweiter Körperteilabschnitte als Kniegelenk
31b erstes Körperteilabschnittsmodell als Oberschenkel
32b erstes Körperteilabschnittsmodell als Unterschenkel
33b zweites Körperteilabschnittsmodell als Kniegelenk
50 Kopf
50a erster Körperteilabschnitt
51 Schulterbereich
51a erster Körperteilabschnitt
52 Hals
52a zweiter Körperteilabschnitt
100 Orthopädietechnische Versorgung

## Patentansprüche

1. Verfahren zum Erstellen eines digitalen 3D-Modells eines Körperteils eines Patienten, das wenigstens einen ersten Körperteilabschnitt (21) hat, der an wenigstens einen gelenkigen zweiten Körperteilabschnitt (22) angrenzt, sodass der erste Körperteilabschnitt (21) mittels des gelenkigen zweiten Körperteilabschnitts (22) bewegbar ist, **dadurch gekennzeichnet, dass** das Verfahren die folgenden durch eine digitale Datenverarbeitungseinrichtung (12) ausgeführten Schritte umfasst:
- Bereitstellen von digitalen, kamerabasierten Bilddaten, die das digital zu modellierende Körperteil des Patienten aus verschiedenen Aufnahmerichtungen beinhalten,
- Identifizieren des wenigstens einen ersten Körperteilabschnitts (21) aus den bereitgestellten digitalen Bilddaten und Erstellen eines ersten 3D-Körperteilabschnittsmodell (31b,32b) für den identifizierten ersten Körperteilabschnitt (21) basierend auf den bereitgestellten digitalen Bilddaten und hierzu bereitgestellten Tiefeninformationen des identifizierten ersten Körperteilabschnitts (21),
- Identifizieren des wenigstens einen gelenkigen zweiten Körperteilabschnitts (22) aus den bereitgestellten digitalen Bilddaten, und
- Erstellen des digitalen 3D-Modells des zu modellierenden Körperteils in Abhängigkeit von dem erstellten ersten 3D-Körperteilabschnittsmodell (31b,32b) und dem identifizierten gelenkigen zweiten Körperteilabschnitts (22).

2. Verfahren nach Anspruch 1, **dadurch gekennzeichnet, dass** ein zweites 3D-Körperteilabschnittsmodell (33b) für den identifizierten gelenkigen zweiten Körperteilabschnitt (22) basierend auf den bereitgestellten digitalen Bilddaten und hierzu bereitgestellten Tiefeninformationen des identifizierten gelenkigen zweiten Körperteilabschnitts (22) erstellt wird, wobei das digitale 3D-Modell des zu modellierenden Körperteils in Abhängigkeit von dem erstellten ersten 3D-Körperteilabschnittsmodell (31b,32b) und dem erstellten zweiten 3D-Körperteilabschnittsmodell (33b) erstellt wird.

3. Verfahren nach Anspruch 1 oder 2, **dadurch gekennzeichnet, dass** wenigstens zwei Körperteilabschnitte (21, 22) weiterhin in Abhängigkeit von einer Beziehung zueinander identifiziert werden.

4. Verfahren nach einem der vorhergehenden Ansprüche, **dadurch gekennzeichnet, dass** der gelenkige zweite Körperteilabschnitt (22) in Abhängigkeit von einem Bewegungsraum des Gelenkes oder dass der erste Körperteilabschnitt (21) in Abhängigkeit von einem Bewegungsraum eines an dem ersten Körperteilabschnitt (21) angrenzenden Gelenkes oder des identifizierten gelenkigen zweiten Körperteilabschnittes (22) identifiziert wird.

5. Verfahren nach einem der vorhergehenden Ansprüche, **dadurch gekennzeichnet, dass** die Körperteilabschnitte (21, 22) des zu modellierenden Körperteils in einem gemeinsamen Scanvorgang erfasst werden, wobei das mindestens eine Körperteilabschnittsmodell (31b,32b,33b) separat erstellt wird.

6. Verfahren nach einem der vorhergehenden Ansprüche, **dadurch gekennzeichnet, dass** ein maschinelles Lernsystem bereitgestellt wird, welches eine Korrelation von digitalen Bilddaten als Eingabedaten mit der jeweiligen Identifikation des entsprechenden Körperteilabschnittes (21,22) als Ausgabedaten gelernt hat, wobei zur Identifikation die bereitgestellten Bilddaten als Eingabedaten in das maschinelle Lernsystem eingegeben werden und daraufhin eine Identifikation des entsprechenden Körperteilabschnittes (21,22) als Ausgabedaten erhalten wird.

7. Verfahren nach Anspruch 6, **dadurch gekennzeichnet, dass** das maschinelle Lernsystem ein künstliches neuronales Netz ist.

8. Verfahren nach einem der vorhergehenden Ansprüche, **dadurch gekennzeichnet, dass** die digitalen, kamerabasierten Bilddaten kontinuierlich der digitalen Datenverarbeitungseinrichtung (12) bereitgestellt werden.

9. Verfahren nach Anspruch 8, **dadurch gekennzeichnet, dass** basierend auf den kontinuierlich bereitgestellten Bilddaten kontinuierlich das 3D-Modell des Körperteils erstellt wird.

10. Verfahren nach einem der vorhergehenden Ansprüche, **dadurch gekennzeichnet, dass** mittels eines mobilen Bildsensors die digitalen, kamerabasierten Bilddaten des zu modellierenden Körperteils des Patienten aufgenommen und der digitalen Datenverarbeitungseinrichtung (12) zum Erstellen des 3D-Körperteilmodells bereitgestellt werden.

11. Verfahren nach Anspruch 10, **dadurch gekennzeichnet, dass** der mobile Bildsensor ein handgeführter Bildsensor ist.

12. Computerprogramm mit Programmcodemitteln eingerichtet zur Durchführung des Verfahrens nach einem der vorhergehenden Ansprüche, wenn das Computerprogramm auf einer Datenverarbeitungsanlage ausgeführt wird.

13. Verfahren zum Einstellen oder Herstellen einer orthopädietechnischen Versorgung (100) eines mit der orthopädietechnischen Versorgung (100) ausgestatteten Patienten, mit den Schritten:
- Erstellen eines 3D-Körperteilmodells des Körperteils, dass mit der orthopädietechnischen Versorgung (100) ausgestattet werden soll, gemäß dem Verfahren nach einem der Ansprüche 1 bis 11, und
- Einstellen oder Herstellen der orthopädietechnischen Versorgung (100) unter Verwendung des 3D-Körperteilmodells.

## Claims

1. Method for creating a digital 3D model of a patient's body part having at least a first body part section (21) adjacent to at least a second articulated body part section (22) such that the first body part section (21) is movable by means of the second articulated body part section (22), **characterized in that** the method comprises the following steps performed by a digital data processing device (12):
- providing digital, camera-based image data comprising the patient's body part to be digitally modeled from various imaging directions,
- identifying the at least one first body part section (21) from the provided digital image data and creating a first 3D body part section model (31b, 32b) for the identified first body part section (21) based on the provided digital image data and depth information provided for the identified first body part section (21),
- identifying the at least one articulated second body part section (22) from the provided digital image data, and
- creating the digital 3D model of the body part to be modeled based on the created first 3D body part section model (31b, 32b) and the identified articulated second body part section (22).

2. Method according to claim 1, **characterized in that** a second 3D body part section model (33b) for the identified articulated second body part section (22) is created based on the provided digital image data and depth information provided for the identified articulated second body part section (22), wherein the digital 3D model of the body part to be modeled is created based on the created first 3D body part section model (31b, 32b) and the created second 3D body part section model (33b).

3. Method according to claim 1 or 2, **characterized in that** at least two body part sections (21, 22) are further identified based on a relationship between them.

4. Method according to any of the preceding claims, **characterized in that** the articulated second body part section (22) is identified based on a range of motion of the joint, or that the first body part section (21) is identified based on a range of motion of a joint adjacent to the first body part section (21) or of the identified articulated second body part section (22).

5. Method according to any one of the preceding claims, **characterized in that** the body part sections (21, 22) of the body part to be modeled are captured in a single scanning process, wherein the at least one body part section model (31b, 32b, 33b) is created separately.

6. Method according to one of the preceding claims, **characterized in that** a machine learning system is provided which has learned a correlation between digital image data as input data and the respective identification of the corresponding body part section (21, 22) as output data, wherein, for the identification, the provided image data is input into the machine learning system as input data, and an identification of the corresponding body part section (21, 22) is then obtained as output data.

7. Method according to claim 6, **characterized in that** the machine learning system is an artificial neural network.

8. Method according to one of the preceding claims, **characterized in that** the digital, camera-based image data is continuously provided to the digital data processing device (12).

9. Method according to claim 8, **characterized in that** the 3D model of the body part is continuously generated based on the continuously provided image data.

10. Method according to one of the preceding claims, **characterized in that** the digital, camera-based image data of the patient's body part to be modeled is captured by means of a mobile image sensor and provided to the digital data processing device (12) for creating the 3D body part model.

11. Method according to claim 10, **characterized in that** the mobile image sensor is a handheld image sensor.

12. Computer program comprising program code means configured to perform the method according to any one of the preceding claims when the computer program is executed on a data processing system.

13. Method for adjusting or manufacturing an orthopedic device (100) for a patient fitted with the orthopedic device (100), comprising the steps of:
- creating a 3D body part model of the body part to be fitted with the orthopedic device (100) according to the method of any one of claims 1 to 11, and
- adjusting or manufacturing the orthopedic device (100) using the 3D body part model.

## Revendications

1. Procédé de création d'un modèle 3D numérique d'une partie du corps d'un patient, comportant au moins une première portion de partie du corps (21) adjacente à au moins une deuxième portion de partie du corps articulée (22), de sorte que la première portion de partie du corps (21) est mobile grâce à la deuxième portion de partie du corps articulée (22),
**caractérisé en ce que** le procédé comprend les étapes suivantes, exécutées par un dispositif numérique de traitement de données (12) :
- fournir des données d'image numériques issues d'une caméra, qui représentent la partie du corps du patient à modéliser numériquement sous différents angles de prise de vue,
- identifier ladite au moins une première portion de partie du corps (21) à partir des données d'image numériques fournies, et créer un premier modèle 3D de la portion de partie du corps (31b, 32b) pour la première portion de partie du corps identifiée (21) sur la base des données d'image numériques fournies et des informations de profondeur fournies à cet effet concernant la première portion de partie du corps identifiée (21),
- identifier ladite au moins une deuxième portion de partie du corps articulée (22) à partir des données d'image numériques fournies, et
- créer le modèle 3D numérique de la partie du corps à modéliser en fonction du premier modèle 3D de la portion de partie du corps (31b, 32b) créé et de la deuxième portion de partie du corps articulée (22) identifiée.

2. Procédé selon la revendication 1,
**caractérisé en ce qu'**un deuxième modèle 3D de la portion de partie du corps (33b) pour la deuxième portion de partie du corps articulée (22) identifiée est créé sur la base des données d'image numériques fournies et des informations de profondeur fournies à cet effet concernant la deuxième portion de partie du corps articulée (22) identifié, le modèle 3D numérique de la partie du corps à modéliser étant créé en fonction du premier modèle 3D de la portion de partie du corps (31b, 32b) créé et du deuxième modèle 3D de la portion de partie du corps (33b) créé.

3. Procédé selon la revendication 1 ou 2,
**caractérisé en ce qu'**au moins deux portions de partie du corps (21, 22) sont en outre identifiés en fonction d'une relation entre elles.

4. Procédé selon l'une des revendications précédentes,
**caractérisé en ce que** la deuxième portion de partie du corps articulée (22) est identifiée en fonction d'un espace de mouvement de l'articulation, ou **en ce que** la première portion de partie du corps (21) est identifiée en fonction d'un espace de mouvement d'une articulation adjacente à la première portion de partie du corps (21) ou de la deuxième portion de partie du corps articulée (22) identifiée.

5. Procédé selon l'une des revendications précédentes,
**caractérisé en ce que** les portions de partie du corps (21, 22) de la partie de corps à modéliser sont capturées au cours d'un processus de balayage commun, ledit au moins un modèle de la portion de partie du corps (31b, 32b, 33b) étant créé séparément.

6. Procédé selon l'une des revendications précédentes,
**caractérisé en ce qu'**il est prévu un système d'apprentissage automatique qui a appris à établir une corrélation entre des données d'image numériques, en tant que données d'entrée, et l'identification respective de la portion de partie du corps (21, 22) correspondante, en tant que données de sortie, les données d'image fournies étant saisies en tant que données d'entrée dans le système d'apprentissage automatique pour l'identification, puis une identification de la portion de partie du corps (21, 22) correspondante étant obtenue en tant que données de sortie.

7. Procédé selon la revendication 6,
**caractérisé en ce que** le système d'apprentissage automatique est un réseau neuronal artificiel.

8. Procédé selon l'une des revendications précédentes,
**caractérisé en ce que** les données d'image numériques issues de la caméra sont fournies en continu au dispositif numérique de traitement de données (12).

9. Procédé selon la revendication 8,
**caractérisé en ce que** le modèle 3D de la partie du corps est créé en continu sur la base des données d'image fournies en continu.

10. Procédé selon l'une des revendications précédentes,
**caractérisé en ce que** les données d'image numériques issues d'une caméra de la partie du corps du patient à modéliser sont enregistrées à l'aide d'un capteur d'images mobile et sont fournies au dispositif numérique de traitement de données (12) pour la création du modèle 3D de la partie du corps.

11. Procédé selon la revendication 10,
**caractérisé en ce que** le capteur d'images mobile est un capteur d'images portatif.

12. Programme informatique comprenant des moyens de code de programme agencés pour mettre en œuvre le procédé selon l'une des revendications précédentes, lorsque le programme informatique est exécuté sur un système de traitement de données.

13. Procédé d'ajustement ou de fabrication d'un équipement orthopédique (100) pour un patient équipé dudit équipement orthopédique (100), comprenant les étapes consistant à :
- créer un modèle 3D de la partie du corps censée être équipée de l'équipement orthopédique (100), par le procédé selon l'une des revendications 1 à 11, et
- ajuster ou fabriquer l'équipement orthopédique (100) à l'aide du modèle 3D de la partie du corps.
